# NOUVEAU FASCICULE DE BREVET EUROPEEN

(11) **EP 1 160 005 B2**
(45) Date de publication et mention de la décision concernant l'opposition: **07.10.2009**
(45) Mention de la délivrance du brevet: 07.08.2002
(21) Numéro de dépôt: 01401040.9
(22) Date de dépôt: 24.04.2001
(51) Int. Cl.: B01F 17/00, A61K 8/00

(54) **Nanoémulsion à base de polymères anioniques, et ses utilisations notamment dans les domaines cosmétique, dermatologique, pharmaceutique et/ou ophthalmologique**
Nanoemulsion auf Basis anionischer Polymeren, und deren Verwendungen insbesondere in Kosmetik, Dermatologie, Pharmazeutik und/oder Ophthalmologie
Nanoemulsion based on anionic polymers, and uses thereof especially in the cosmetic, dermatological, pharmaceutical and/or ophthalmic fields

(30) Priorité: 22.05.2000 FR 0006511
(43) Date de publication de la demande: 05.12.2001
(73) Titulaire: L'ORÉAL, 75008 Paris (FR)
(72) Inventeur: Sonneville-Aubrun, Odile, 92160 Antony (FR); Simonnet, Jean-Thierry, 75011 Paris (FR)
(74) Mandataire: Dossmann, Gérard

(56) Documents cités:
- EP-A- 0 842 652
- WO-A-96/128132

## Description

La présente invention a trait à une nanoémulsion, comprenant au moins un lipide amphiphile non ionique et/ou anionique et au moins un polymère anionique particulier non réticulé comportant au moins une chaîne hydrophobe, ainsi qu'à l'utilisation de ladite nanoémulsion en application topique notamment dans les domaines cosmétique et dermatologique, et dans les domaines pharmaceutique et/ou ophtalmologique.

Les émulsions huile-dans-eau (H/E) sont bien connues dans le domaine de la cosmétique et de la dermatologie, notamment pour la préparation de produits cosmétiques tels que des laits, des crèmes, des toniques, des sérums, des eaux de toilette.

Les nanoémulsions sont des émulsions H/E caractérisées par une taille des globules huileux inférieure à 100 nm, les globules huileux étant stabilisés par une couronne de lipides amphiphiles pouvant éventuellement former une phase cristal liquide de type lamellaire, situés à l'interface huile/phase aqueuse. La transparence de ces émulsions provient de la petite taille des globules huileux, petite taille obtenue grâce à l'utilisation d'une énergie mécanique et notamment d'un homogénéisateur haute pression. Les nanoémulsions sont à différencier des microémulsions de par leur structure. En effet, les microémulsions sont des dispersions thermodynamiquement stables constituées de micelles de lipide(s) amphiphile(s) gonflées par de l'huile. De plus, les microémulsions ne nécessitent pas d'énergie mécanique importante pour être réalisées; elles se forment spontanément par simple mise en contact des constituants. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit et leur stabilité en température très limitée.

Les nanoémulsions comprennent un ou plusieurs lipide(s) amphiphile(s). Par lipide amphiphile, on entend ici toutes molécules ayant une structure bipolaire, c'est-à-dire comportant au moins une partie hydrophobe et au moins une partie hydrophile et ayant la propriété de réduire la tension superficielle de l'eau (γ< 55mN/m) et de réduire la tension interfaciale entre l'eau et une phase huileuse. Les synonymes de lipide amphiphile sont par exemple : tensioactif, agent de surface, émulsionnant.

Les documents EP-A-728 460 et EP-A-780 114 décrivent des nanoémulsions à base de lipides amphiphiles non ioniques liquides ou de tensioactifs siliconés. Des nanoémulsions sont également décrites dans les documents FR-A-2,787,026, FR-A-2,787,027, FR-A-2,787,325, FR-A-2,787,326, FR-A-2,787,703, FR-A-2,787,728, et dans les demandes FR9900031, FR9900408 et FR9901178.

Pour que les nanoémulsions telles que décrites dans ces documents puissent être utilisées comme laits ou crèmes, et notamment dans le domaine du soin, il faut les rendre plus épaisses et donc augmenter leur viscosité. Il existe deux moyens pour augmenter la viscosité d'une nanoémulsion. Le premier moyen consiste à augmenter la fraction de la phase huileuse dispersée. En effet, à partir de 22 % en poids de phase huileuse par rapport au poids total de la composition, on constate généralement que la viscosité augmente en fonction du taux d'huile. Cette méthode, décrite dans les demandes citées ci-dessus, permet d'obtenir des compositions épaisses, transparentes et stables. Toutefois, la contrainte d'une telle méthode d'épaississement des nanoémulsions est l'obligation d'avoir un taux d'huile élevé, ce qui n'est pas toujours souhaité car les formules obtenues sont plus riches (taux élevé de phase grasse) et la gamme de viscosité est plus étroite.

Le second moyen pour augmenter la viscosité d'une nanoémulsion consiste à ajouter à la nanoémulsion un polymère hydrophile qui, par gélification de la phase continue aqueuse, va augmenter la viscosité de l'ensemble, et ce, même avec de faibles taux d'huile. Dans les demandes citées ci-dessus, il est envisagé l'adjonction de polymères hydrophiles tels que les dérivés de cellulose, les dérivés d'algues, les gommes naturelles et les polymères synthétiques comme les polymères et copolymères d'acides carboxyvinyliques, par exemple les Carbopols. Malheureusement, les produits obtenus ne sont pas toujours transparents ; ou alors dans des conditions de concentration faible en polymère, ce qui limite leur effet.

Ainsi, il subsiste le besoin d'un système épaississant permettant d'épaissir de manière convenable une composition sous forme de nanoémulsion huile-dans-eau, sans influer sur les propriétés cosmétiques desdites compositions, notamment sans influer sur le caractère transparent de la nanoémulsion, et ce quel que soit le taux d'huile que l'on souhaite utiliser.

La demanderesse a découvert, de façon inattendue, que l'on pouvait épaissir les nanoémulsions, avec des polymères anioniques particuliers non réticulés de préférence hydrosolubles ou hydrodispersibles, comportant au moins une chaîne hydrophobe.

La présente invention a pour objet une nanoémulsion huile-dans-eau comportant une phase huileuse dispersée dans une phase aqueuse, dont les globules d'huile ont une taille moyenne inférieure à 100 nm, caractérisée en ce qu'elle comprend
(1) au moins un lipide amphiphile choisi parmi les lipides amphiphiles non ioniques, les lipides amphiphiles anioniques et leurs mélanges, le ou les lipides amphiphiles anioniques étant choisis parmi les esters mixtes d'acide gras ou d'alcool gras, d'acide carboxylique et de glycérol, les citrates d'alkyléther, les alkényl succinates, les esters gras d'acide phosphoriques et leurs mélanges, et
(2) au moins un polymère anionique non réticulé comportant au moins une chaîne hydrophobe choisi parmi les copolymères d'acide acrylique ou méthacrylique, les copolymères d'acide 2-acrylamido-2-méthylpropane sulfonique et leurs mélanges, et en ce que le rapport pondéral de la quantité de phase huileuse sur la quantité de lipide amphiphile va de 1,2 à 10 de 2 à 10, mieux de 2 à 6 et encore mieux de 3 à 6.

Les nanoémulsions selon l'invention ont généralement un aspect transparent à bleuté. Leur transparence se mesure par un coefficient de transmittance à 600 nm allant de 10 à 90 % ou bien par une turbidité allant de 60 à 600 NTU et de préférence de 70 à 300 NTU, turbidité mesurée au turbidimètre portatif HACH - Modèle 2100 P.

Les globules d'huile des nanoémulsions de l'invention ont une taille moyenne (taille moyenne en nombre) inférieure à 100 nm et de préférence allant de 20 à 80 nm et plus préférentiellement de 40 à 60 nm. La diminution de la taille des globules permet de favoriser la pénétration des actifs dans les couches superficielles de la peau (effet véhicule).

Les nanoémulsions conformes à l'invention sont préparées de préférence à des températures allant de 4 à 45°C et sont ainsi compatibles avec des actifs thermosensibles.

Les polymères anioniques non réticulés utilisés selon la présente invention sont de préférence hydrosolubles ou hydrodispersibles, c'est-à-dire qu'ils sont solubles dans l'eau à un pH supérieur à 3,5. Ils ont de préférence un poids moléculaire allant de 10.000 à 2.000.000. Ces polymères permettent d'augmenter la viscosité des nanoémulsions fluides (5 cP) d'au moins d'un facteur 10. Ajoutés à une nanoémulsion, ils permettent d'obtenir des compositions transparentes et stables, constituant des crèmes. On entend par « lait » ou « crème » des compositions ayant une viscosité allant de 0,5 à 150 Poises (soit 0,05 Pa.s à 15 Pa.s) mesurée à 25°C avec Rheomat 180 au mobile 3, 4 ou 5 (selon la gamme de viscosité), à 200 s⁻¹.

Aussi, un autre objet de l'invention est un procédé pour épaissir une nanoémulsion huile-dans-eau ayant des globules d'huile dont la taille moyenne est inférieure à 100 nm, consistant à ajouter à ladite nanoémulsion un polymère anionique comportant au moins une chaîne hydrophobe.

La ou les chaînes hydrophobes du polymère anionique non réticulé utilisé selon l'invention sont notamment des chaînes hydrocarbonées linéaires ou ramifiées, saturées ou insaturées, ayant de 6 à 30 atomes de carbone, telles que alkyle, arylalkyle, alkylaryle, alcoylène ; des groupements divalents cycloaliphatiques tels que notamment méthylènedicyclohexyl et isophorone ; ou des groupements divalents aromatiques tels que phénylène.

Parmi les polymères anioniques non réticulés utilisés dans la présente invention, les copolymères d'acide acrylique ou méthacrylique sont préférés. On entend par « copolymères » aussi bien les copolymères obtenus à partir de deux sortes de monomères que ceux obtenus à partir de plus de deux sortes de monomères tels que les terpolymères obtenus à partir de trois sortes de monomères.

Les polymères anioniques non réticulés utilisés de préférence dans l'invention sont obtenus par copolymérisation d'un monomère (a) choisi parmi les acides acrylique ou méthacrylique (monomère a') et l'acide 2-acrylamido 2-méthylpropane sulfonique (monomère a"), avec un monomère (b) à insaturation éthylénique non tensioactif différent de (a) et/ou un monomère (c) à insaturation éthylénique issu de la réaction d'un monomère acrylique à insaturation α,β-monoéthylénique ou d'un monomère isocyanate à insaturation monoéthylénique avec un composant amphiphile non ionique monohydrique ou avec une amine grasse primaire ou secondaire.

Ainsi, les polymères anioniques non réticulés utilisés dans la composition de l'invention peuvent être obtenus par deux voies de synthèses :
- soit par copolymérisation des monomères (a') et (c), ou (a'), (b) et (c), ou (a") et (c), ou (a"), (b) et (c),
- soit par modification (et notamment estérification ou amidification) d'un copolymère formé à partir des monomères (a') ou à partir des monomères (a') et (b), ou (a") et (b), par un composé amphiphile non ionique monohydrique ou une amine grasse primaire ou secondaire.

Comme copolymères d'acide 2-acrylamido 2-méthylpropane sulfonique, on peut citer notamment ceux décrits dans l'article « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » et dans les demandes EP-A-0 750 899 et EP-A-1,069,172.

Le copolymère peut contenir un monomère (b) à insaturation monoéthylénique qui n'a pas de propriété tensioactive. Les monomères préférés sont ceux qui donnent des polymères insolubles dans l'eau lorsqu'ils sont homopolymérisés. Ils peuvent être choisis par exemple parmi les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont l'acrylate de méthyle et l'acrylate d'éthyle. Les autres monomères pouvant être utilisés sont par exemple le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Toutefois, des monomères qui contiennent des groupes réactifs sous l'action de la chaleur comme l'acrylate d'hydroxyéthyle peuvent éventuellement être utilisés.

Le monomère (c) est obtenu par réaction d'un monomère acrylique à insaturation α,β-monoéthylénique tel que (a), ou d'un monomère isocyanate à insaturation monoéthylénique, avec un composé amphiphile non ionique monohydrique ou une amine grasse primaire ou secondaire.

Les composés amphiphiles non ioniques monohydriques ou les amines grasses, primaires ou secondaires, utilisés pour obtenir le monomère non-ionique (c) sont bien connus. Les composés amphiphiles non ioniques monohydriques sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du composé amphiphile.

Les composés amphiphiles non ioniques monohydriques préférés sont des composés ayant la formule (I) suivante :

R-(OCH₂CHR')ₘ-(OCH₂CH₂)ₙ-OH (I)

dans laquelle R est choisi parmi les groupes alkyle ou alcoylène comportant de 6 à 30 atomes de carbone et les groupes alkylaryle ayant des radicaux alkyle comportant de 8 à 30 atomes de carbone, R' est choisi parmi les groupes alkyle comportant de 1 à 4 atomes de carbone, n est un nombre moyen allant d'environ 1 à 150 et m est un nombre moyen allant d'environ 0 à 50, à la condition que n soit au moins aussi grand que m.

De préférence, dans les composés de formule (I), le groupe R est choisi parmi les groupes alkyle comportant de 12 à 26 atomes de carbone et les groupes alkyl-(C₈-C₁₃)-phényle ; le groupe R' est le groupe méthyle ; m = 0 et n = 1 à 25.

Les amines grasses primaires et secondaires préférées sont constituées d'une ou deux chaînes alkyles comportant de 6 à 30 atomes de carbone.

Le monomère utilisé pour former le monomère uréthane non-ionique (c) peut être choisi parmi des composés très variés. On peut utiliser tout composé contenant une insaturation copolymérisable telle qu'une insaturation acrylique, méthacrylique ou allylique. Le monomère (c) peut être obtenu notamment à partir d'un isocyanate à insaturation monoéthylénique tel que notamment l'α,α-diméthyl-m-isopropényl-benzyl-isocyanate.

Le monomère (c) peut être en particulier choisi parmi les acrylates, méthacrylates ou itaconates d'alcool gras en C₆-C₃₀ oxyéthyléné (1 à 50 OE), tels que le steareth-20 méthacrylate, le méthacrylate de béhényle oxyéthyléné (25 OE), l'itaconate de mono-cétyle oxyéthyléné (20 OE), l'itaconate de mono-stéaryle oxyéthyléné (20 OE), l'acrylate modifié par des alcools en C12-C24 polyoxyéthylénés (25 OE), et parmi les diméthyl m-isopropénylbenzylisocyanates d'alcool gras en C₆-C₃₀ oxyéthyléné (1 à 50 OE), tel que notamment le diméthyl m-isopropénylbenzylisocyanate d'alcool béhénylique oxyéthyléné.

Selon un mode particulier de réalisation de l'invention, le polymère anionique non réticulé est choisi parmi les terpolymères acryliques obtenus à partir de (a) un acide carboxylique à insaturation α,β-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique.

Comme polymères anioniques non réticulés comportant au moins une chaîne hydrophobe, pouvant être utilisés dans la nanoémulsion de l'invention, on peut citer notamment le terpolymère acide acrylique/acrylate d'éthyle/acrylate d'alkyle, tel que le produit en dispersion aqueuse à 30 % commercialisé sous la dénomination Acusol 823 par la société Rohm & Haas ; le copolymère acrylates/steareth-20 methacrylate tel que le produit commercialisé sous la dénomination Aculyn 22 par la société Rohm & Haas ; le terpolymère acide (méth)acrylique / acrylate d'éthyle / méthacrylate de béhényle oxyéthyléné (25 OE), tel que le produit en émulsion aqueuse commercialisé sous la dénomination Aculyn 28 par la société Rohm & Haas ; le copolymère acide acryliquelitaconate de mono-cétyle oxyéthyléné (20 OE), tel que le produit en dispersion aqueuse à 30 % commercialisé sous la dénomination Structure 3001 par la société National Starch ; le copolymère acide acrylique/itaconate de mono-stéaryle oxyéthyléné (20 OE) tel que le produit en dispersion aqueuse à 30 % commercialisé sous la dénomination Structure 2001 par la société National Starch ; le copolymère acrylates/acrylate modifié par des alcools en C12-C24 polyoxyéthylénés (25 OE), tel que le latex à 30-32 % de copolymère, commercialisé sous la dénomination Synthalen W2000 par la société 3V SA ; le terpolymère acide méthacrylique/acrylate de méthyle/diméthylmétaisopropényl benzylisocyanate d'alcool béhénylique éthoxylé, tel que le produit en dispersion aqueuse à 24 % et comportant 40 groupes oxyéthylénés, décrit dans le document EP-A-0 173 109.

Selon l'invention, le ou les polymères anioniques peuvent représenter de 0,1 à 10 % en poids, de préférence de 0,2 à 5 % en poids et plus particulièrement de 1 à 5 % en poids par rapport au poids total de la composition.

L'addition de neutralisants peut s'avérer utile pour augmenter la solubilité des polymères dans l'eau. On peut alors utiliser tout neutralisant connu, et en particulier on peut le choisir parmi les bases inorganiques telles que la soude, la potasse, l'ammoniac, et parmi les bases organiques telles que la mono-, di- et tri-éthanolamine, l'aminométhylpropanediol-1,3, la N-méthylglucamine, les acides aminés basiques comme l'arginine et la lysine, et leurs mélanges. Le pH des compositions selon l'invention doit être supérieur à 4, et va de préférence de 5 à 8 et mieux de 5 à 7. La quantité de neutralisant dépend du polymère utilisé et des autres constituants de la formule. Elle peut aller par exemple de 0,01 à 5 % et mieux de 0,05 à 5 % du poids total de la composition.

Les polymères anioniques non réticulés utilisés selon l'invention peuvent épaissir tout type de nanoémulsions, et notamment les nanoémulsions décrites dans les documents EP-A-705 593, EP-A-728 460, EP-A-780 114, FR-A-2,787,026, FR-A-2,787,027, FR-A-2,787,325, FR-A-2,787,326, FR-A-2,787,703, FR-A-2,787,728, FR9900031, FR9900408 et FR9901178.

Ces nanoémulsions comprennent au moins un lipide amphiphile choisi parmi les lipides amphiphiles non ioniques, les lipides amphiphiles anioniques tels que définis ci-dessus et leurs mélanges.

Les lipides amphiphiles non ioniques de l'invention sont préférentiellement choisis parmi:
1/ les tensioactifs siliconés,
2/ les lipides amphiphiles liquides à température inférieure ou égale à 45°C, choisis parmi les esters d'au moins un polyol et d'au moins un acide gras comportant au moins une chaîne alkyle en C₈-C₂₂, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol et,
3/ les esters d'acide gras et de sucre et les éthers d'alcool gras et de sucre,
4/ les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés,
5/ Les copolymères blocs d'oxyde d'éthylène (A) et d'oxyde de propylène (B), et leurs mélanges.

1/ Les tensioactifs siliconés utilisables selon l'invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5,364,633 et US-A-5,411,744.
   De préférence, le tensioactif siliconé utilisé selon la présente invention est un composé de formule (II) : 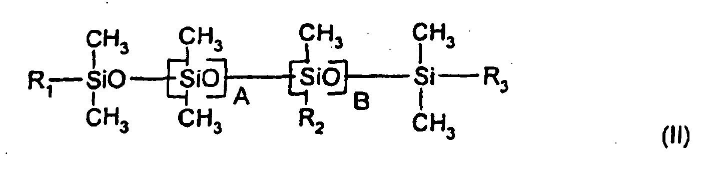 dans laquelle :
   R₁, R₂, R₃, indépendamment les uns des autres, représentent un radical alkyle en C₁-C₆ ou un radical -(CH₂)ₓ-(OCH₂CH₂)_{y}-(OCH₂CH₂CH₂)_{z}-OR₄, au moins un radical R₁, R₂ ou R₃ n'étant pas un radical alkyle ; R₄ étant un hydrogène, un radical alkyle ou un radical acyle ;
   A est un nombre entier allant de 0 à 200 ;
   B est un nombre entier allant de 0 à 50 ; à la condition que A et B ne soient pas égaux à zéro en même temps ;
   x est un nombre entier allant de 1 à 6 ;
   y est un nombre entier allant de 1 à 30 ;
   z est un nombre entier allant de 0 à 5.
   Selon un mode de réalisation préféré de l'invention, dans le composé de formule (I), le radical alkyle est un radical méthyle, x est un nombre entier allant de 2 à 6 et y est un nombre entier allant de 4 à 30.
   On peut citer, à titre d'exemple de tensioactifs siliconés de formule (II), les composés de formule (III) : 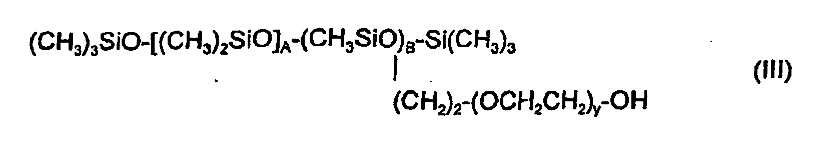 dans laquelle A est un nombre entier allant de 20 à 105, B est un nombre entier allant de 2 à 10 et y est un nombre entier allant de 10 à 20.
   On peut également citer à titre d'exemple de tensioactifs siliconés de formule (II), les composés de formule (IV) :

   H-(OCH₂CH₂)_{y}-(CH₂)₃-[(CH₃)₂SiO]_{A}-(CH₂)₃-(OCH₂CH₂)_{y}-OH (IV)

   dans laquelle A' et y sont des nombres entiers allant de 10 à 20.
   On peut utiliser notamment comme tensioactifs siliconés, ceux commercialisés par la société Dow Coming sous les dénominations DC 5329, DC 7439-146, DC 2-5695 et Q4-3667. Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.
   Le composé Q4-3667 est un composé de formule (IV) où A est 15 et y est 13.
2/ Les lipides amphiphiles liquides à température inférieure ou égale à 45°C peuvent être notamment choisis parmi :
   - l'isostéarate de polyéthylèneglycol de poids moléculaire 400, vendu sous la dénomination PEG 400 par la société UNICHEMA ;
   - l'isostéarate de diglycéryle, vendu par la société SOLVAY ;
   - le laurate de polyglycérol comportant 2 unités de glycérol (polyglyceryl-2 laurate), vendu sous la dénomination Diglycerin-monolaurate par la société SOLVAY ;
   - l'oléate de sorbitan, vendu sous la dénomination SPAN 80 par la société ICI ;
   - l'isostéarate de sorbitan, vendu sous la dénomination NIKKOL SI 10R par la société NIKKO ;
   - le cocoate d'α-butylglucoside ou le caprate d'α-butylglucoside commercialisés par la société ULICE.
3/ Les esters d'acide gras et de sucre, utilisables comme lipides amphiphiles non ioniques dans la nanoémulsion selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.
   Les acides gras en C₈-C₂₂ ou en C₁₄-C₂₂ formant le motif gras des esters utilisables dans la nanoémulsion de l'invention comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.
   On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'O-hexadécanoyle-6-D-glucoside de méthyle et l'O-hexadécanoyle-6-D-maltoside de méthyle.
   Les éthers d'alcool gras et de sucre, utilisables comme lipides amphiphiles non ioniques dans la nanoémulsion selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.
   Les alcools gras en C₈-C₂₂ ou en C₁₄-C₂₂ formant le motif gras des éthers utilisables dans la nanoémulsion de l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanyle, et leurs mélanges tels que cétéaryle.
   A titre d'exemple d'éthers d'alcool gras et de sucre, on peut citer les alkylpolyglucosides tels que le décylglucoside et le laurylglucoside commercialisés par exemple par la société Henkel sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidylglucoside, par exemple sous la forme du mélange d'alcools arachidique et béhénique et d'arachidylglucoside, commercialisé sous la dénomination Montanov 202 par la société Seppic.
   On utilise plus particulièrement comme lipide amphiphile non ionique de ce type, le monostéarate de sucrose, le distéarate de sucrose, le tristéarate de sucrose et leurs mélanges, le distéarate de méthyl glucose et de polyglycérol-3 et les alkylpolyglucosides.
4/ Les esters gras de glycérol, utilisables comme lipides amphiphiles non ioniques dans la nanoémulsion selon l'invention, solides à une température inférieure ou égale à 45°C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la nanoémulsion de l'invention.
   Ces esters peuvent être notamment choisis parmi les stéarates, béhénates, arachidates, palmitates et leurs mélanges. On utilise de préférence des stéarates et palmitates.
   On peut citer à titre d'exemple de tensioactif utilisable dans la nanoémulsion de l'invention, les monostéarate, distéarate, tristéarate et pentastéarate de décaglycérol (10 unités de glycérol) (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.
   Les esters gras de sorbitan, utilisables comme lipides amphiphiles non ioniques dans la nanoémulsion selon l'invention, solides à une température inférieure ou égale à 45°C, sont choisis notamment dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).
   Ces esters peuvent être notamment choisis parmi les stéarates, béhénates, arachidates, palmitates, et leurs mélanges. On utilise de préférence des stéarates et palmitates.
   On peut citer à titre d'exemple d'ester gras de sorbitan et d'ester gras de sorbitan oxyéthyléné, utilisable dans la nanoémulsion de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.
   Les éthers gras éthoxylés solides à une température inférieure ou égale à 45°C, utilisables comme lipides amphiphiles non ioniques dans la nanoémulsion selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.
   Les esters gras éthoxylés solides à une température inférieure ou égale à 45°C, utilisables comme lipides amphiphiles non ioniques dans la nanoémulsion selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.
5/ Les copolymères blocs d'oxyde d'éthylène (A) et d'oxyde de propylène (B), utilisables comme lipides amphiphiles non ioniques dans la nanoémulsion selon l'invention peuvent être choisis notamment parmi les copolymères blocs de formule (V) :

   HO(C₂H₄O)x(C₃H₆O)y(C₂H₄O)zH (V)

   dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (V) avec x=z=6, y=39 (HLB 2) ; le Poloxamer 282 tel que le produit commercialisé par la société ICI sous la dénomination Pluronic L92 de formule (V) avec x=z=10, y=47 (HLB 6) ; et le Poloxamer 124 tel que le produit commercialisé par la société ICI sous la dénomination Pluronic L44 de formule (V) avec x=z=11, y=21 (HLB 16).

Comme lipides amphiphiles non ioniques, on peut aussi citer les mélanges de tensioactifs non ioniques décrits dans le document EP-A-705593 incorporé ici pour référence.

Parmi les lipides amphiphiles non ioniques, on peut utiliser en particulier :
- l'isostéarate de PEG 400 ou PEG-8 Isostéarate (comportant 8 moles d'oxyde d'éthylène),
- l'isostéarate de diglycéryle,
- le monolaurate de polyglycérol comportant 2 unités de glycérol et les stéarates de polyglycérol comportant 10 unités de glycérol,
- l'oléate de sorbitan,
- l'isostéarate de sorbitan,
et leurs mélanges.

Les lipides amphiphiles anioniques utilisables dans les nanoémulsions de l'invention peuvent être choisis parmi :
1/ les esters mixtes d'acide gras ou d'alcool gras, d'acide carboxylique et de glycérol,
2/ les citrates d'alkyléther,
3/ les alkényl succinates choisis parmi les alkényl succinates alkoxylés, les alkényl succinates de glucose alkoxylés et les alkényl succinates de méthylglucose alkoxylés,
4/ les esters gras d'acide phosphoriques.

1/ Les esters mixtes d'acide gras ou d'alcool gras, d'acide carboxylique et de glycérol, utilisables comme lipides amphiphiles anioniques dans la nanoémulsion selon l'invention peuvent être choisis notamment dans le groupe comprenant les esters mixtes d'acide gras ou d'alcool gras ayant une chaîne alkyle comportant de 8 à 22 atomes de carbone, et d'alpha-hydroxyacide et/ou d'acide succinique, avec la glycérine. L'alpha-hydroxyacide peut être par exemple l'acide citrique, l'acide lactique, l'acide glycolique, l'acide malique et leurs mélanges.
   La chaîne alkyle des acides ou alcools gras dont dérivent les esters mixtes utilisables dans la nanoémulsion de l'invention peut être linéaire ou ramifiée, saturée ou non saturée. Il peut s'agir notamment de chaînes stéarate, isostéarate, linoléate, oléate, béhénate, arachidonate, palmitate, myristate, laurate, caprate, isostéaryle, stéaryle, linoléyle, oléyle, béhényle, myristyle, lauryle, capryle et leurs mélanges.
   On peut citer, à titre d'exemple d'esters mixtes utilisables dans la nanoémulsion de l'invention, l'ester mixte de glycérine et du mélange d'acides citrique, lactique, linoléique et oléique (nom CTFA : Glyceryl citrate/lactate/linoleate/oleate) commercialisé par la société Hüls sous la dénomination Imwitor 375 ; l'ester mixte d'acide succinique et d'alcool isostéarylique avec la glycérine (nom CTFA : Isostéaryl diglycéryl succinate) commercialisé par la société Hüls sous la dénomination Imwitor 780 K ; l'ester mixte d'acide citrique et d'acide stéarique avec la glycérine (nom CTFA : Glyceryl stearate citrate) commercialisé par la société Hüls sous la dénomination Imwitor 370 ; l'ester mixte d'acide lactique et d'acide stéarique avec la glycérine (nom CTFA : Glyceryl stearate lactate) commercialisé par la société Danisco sous la dénomination Lactodan B30 ou Rylo LA30.
2/ Les citrates d'alkyléther utilisables comme lipides amphiphiles anioniques dans la nanoémulsion selon l'invention peuvent être choisis notamment dans le groupe comprenant les monoesters, diesters ou triesters formés par l'acide citrique et au moins un alcool gras oxyéthyléné, comportant une chaîne alkyle linéaire ou ramifiée, saturée ou non saturée, ayant de 8 à 22 atomes de carbone, et comportant de 3 à 9 groupes oxyéthylénés, et leurs mélanges. On peut en effet utiliser un mélange d'un ou plusieurs de ces citrates dans la nanoémulsion de l'invention.
   Ces citrates peuvent être par exemple choisis parmi les mono-, di- et tri-esters d'acide citrique et d'alcool laurique éthoxylé, comportant de 3 à 9 groupes oxyéthylénés, commercialisés par la société Witco sous la dénomination Witconol EC, en particulier le Witconol EC 2129 qui est majoritairement un dilaureth-9 citrate, et le Witconol EC 3129 qui est majoritairement un trilaureth-9 citrate.
   Les citrates d'alkyléther, utilisés comme lipides amphiphiles anioniques sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et tri-éthanolamine, l'aminométhylpropanediol-1,3, la N-méthylglucamine, les acides aminés basiques comme l'arginine et la lysine, et leurs mélanges.
3/ Les alkényl succinates utilisables comme lipides amphiphiles anioniques dans la nanoémulsion de l'invention sont notamment des dérivés éthoxylés et/ou propoxylés et ils sont de préférence choisis parmi les composés de formules (VI)
   ou (VII) :

   HOOC-(HR)C-CH₂-COO-E (VI)

   HOOC-(HR)C-CH₂-COO-E-O-CO-CH₂-C(HR')-COOH (VII)
   dans lesquelles :
   - les radicaux R et R' sont choisis parmi les radicaux alcoylène, linéaires ou ramifiés, comportant de 6 à 22 atomes de carbone,
   - E est choisi parmi les chaînes oxyéthylénées de formule (C₂H₄O)ₙ dans laquelle n va de 2 à 100, les chaînes oxypropylénées de formule (C₃H₆O)ₙ, dans laquelle n' va de 2 à 100, les copolymères statistiques ou séquencés comprenant des chaînes oxyéthylénées de formule (C₂H₄O)ₙ et des chaînes oxypropylénées de formule (C₃H₆O)_{n'} telles que la somme de n et n' va de 2 à 100, les groupements glucoses oxyéthylénés et/ou oxypropylénés comportant en moyenne de 4 à 100 motifs oxyéthylénés et/ou oxypropylénés répartis sur l'ensemble des fonctions hydroxyle, les groupements méthyl glucoses oxyéthylénés et/ou oxypropylénés comportant en moyenne de 4 à 100 motifs oxyéthylénés et/ou oxypropylénés répartis sur l'ensemble des fonctions hydroxyle.
   Dans les formules (VI) et (VII), n et n' sont des valeurs moyennes et ne sont donc pas forcément des entiers. On choisit avantageusement pour n une valeur allant de 5 à 60 et encore plus préférentiellement de 10 à 30.
   Avantageusement, le radical R et/ou R' est choisi parmi les radicaux alcoylène linéaires comportant de 8 à 22 et de préférence de 14 à 22 atomes de carbone. Il peut s'agir par exemple du radical hexadécényl comportant 16 atomes de carbone ou du radical octadécényl comportant 18 atomes de carbone.
   Les composés de formules (VI) et (VII) décrits ci-dessus dans lesquels E est choisi parmi les chaînes oxyéthylénées, les chaînes oxypropylénées et les copolymères comprenant des chaînes oxyéthylénées et des chaînes oxypropylénées, peuvent être préparés conformément à la description qui est donnée dans les documents WO-A-94/00508, EP-A-107199 et GB-A-2131820 incorporés ici pour référence.
   La fonction acide -COOH des lipides amphiphiles anioniques de formules (VI) et (VII) se trouve en général dans la nanoémulsion de l'invention, sous forme neutralisée par un agent de neutralisation, l'agent de neutralisation étant choisi par exemple parmi les bases inorganiques telles que la soude, la potasse, l'ammoniac, et les bases organiques telles que la mono-, di- et tri-éthanolamine, l'aminométhylpropanediol-1.3, la N-méthyl-glucamine, les acides aminés basiques comme l'arginine et la lysine, et leurs mélanges.
   A titre d'exemple de lipide amphiphile anionique de ce type, utilisable dans la nanoémulsion de l'invention, on peut citer l'hexadécényl succinate 18 OE (composé de formule VI avec R=hexadécényl, E=(C₂H₄O)ₙ, n=18), l'hexadécényl succinate 45 OE (composé de formule VI avec R=hexadécényl, E=(C₂H₄O)ₙ, n=45), le dihexadécényl succinate 18 OE (composé de formule VII avec R=R'=hexadécényl, E=(C₂H₄O)ₙ, n=18), le dihexadécényl succinate de glucose 10 OE (composé de formule VII avec R=R'=hexadécényl, E= glucose oxyéthyléné comportant 10 groupes oxyéthylénés), le dihexadécényl succinate de glucose 20 OE (composé de formule VII avec R=R'=hexadécényl, E=glucose oxyéthyléné comportant 20 groupes oxyéthylénés), le dioctadécényl-succinate de méthyl glucose 20 OE (composé de formule VII avec R=R'=octadécényl, E= méthyl glucose oxyéthyléné comportant 20 groupes oxyéthylénés), et leurs mélanges.
4/ Les esters gras d'acide phosphorique, et leurs dérivés oxyéthylénés, utilisables comme lipides amphiphiles anioniques dans la nanoémulsion selon l'invention peuvent être choisis notamment dans le groupe comprenant les esters formés d'acide phosphorique et d'au moins un alcool comportant une chaîne alkyle linéaire ou ramifiée, saturée ou non saturée, ayant de 8 à 22 atomes de carbone et les esters formés d'acide phosphorique et d'au moins un alcool éthoxylé, comportant une chaîne alkyle linéaire ou ramifiée, saturée ou non saturée, ayant de 8 à 22 atomes de carbone et comportant de 2 à 40 groupes oxyéthylénés, leurs sels et leurs mélanges. On peut en effet utiliser un mélange d'un ou plusieurs de ces esters d'acide phosphorique dans la nanoémulsion de l'invention.

Ces esters peuvent être notamment choisis parmi les esters d'acide phosphorique et d'alcools en C9-C15 ou leurs sels, tels que le sel de potassium d'alkyl-C9-15-phosphate commercialisé sous la dénomination Arlatone MAP par la société ICl ; les esters d'acide phosphorique et d'alcools stéarylique et/ou isostéarylique, tels que le phosphate d'alcools stéarylique/isostéarylique (nom CTFA : Octyldecyl phosphate), commercialisé sous la dénomination Hostaphat CG120 par la société Hoechst Celanese ; les esters d'acide phosphorique et d'alcool cétylique, et leurs dérivés oxyéthylénés, tels que le produit commercialisé sous la dénomination Crodafos CES (mélange d'alcool cétéarylique, de dicétyl phosphate et de ceteth-10 phosphate) par la société Croda ; les esters d'acide phosphorique et d'alcool tridécylique, et leurs dérivés oxyéthylénés, tels que le produit commercialisé sous la dénomination Crodafos T10 (nom CTFA : Trideceth-10 Phosphate) par la société Croda. Les dérivés oxyéthylénés d'acide phosphorique et d'alcool gras peuvent être préparés conformément à la description donnée dans la demande de brevet WO-A-96/14145 dont le contenu est incorporé à la présente demande par référence.

Ces esters gras d'acide phosphorique sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et tri-éthanolamine, l'aminométhylpropanediol-1,3, la N-méthylglucamine, les acides aminés basiques comme l'arginine et la lysine, et leurs mélanges.

Selon son caractère plus hydrophile ou plus lipophile, le lipide amphiphile non ionique ou anionique peut être introduit dans la phase aqueuse ou dans la phase huileuse de la nanoémulsion. La quantité de lipide amphiphile va de 0,2 à 15 % en poids et de préférence de 1 à 10 % en poids par rapport au poids total de la nanoémulsion.

On entend ici par "quantité de phase huileuse" la quantité totale des constituants de cette phase sans inclure la quantité de lipide amphiphile.

Selon une forme particulière de réalisation de l'invention, la nanoémulsion de l'invention peut contenir en outre un ou plusieurs lipides amphiphiles anioniques additionnels. Leur ajout, comme additif, peut améliorer encore la stabilité de la dispersion.

Ainsi, les lipides amphiphiles anioniques additionnels pouvant être utilisés dans les nanoémulsions de l'invention sont choisis de préférence parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule (VIII) : 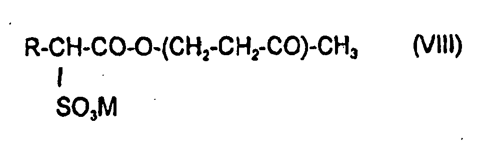
dans laquelle R représente des radicaux alkyle en C₁₆-C₂₂, en particulier les radicaux C₁₆H₃₃ et C₁₈H₃₇ pris en mélange ou séparément et M est un métal alcalin
ou alcalino-terreux tel que le sodium ; et leurs mélanges.

Lorsque la nanoémulsion contient un ou plusieurs lipides amphiphiles additionnels anioniques, ils sont présents dans les nanoémulsions de l'invention, de préférence, dans des concentrations allant de 0,01 à 10 % en poids par rapport au poids total de la nanoémulsion et plus particulièrement de 0,2 à 1 % en poids.

La phase huileuse de la nanoémulsion selon l'invention comprend au moins une huile. Les huiles pouvant être utilisées dans les nanoémulsions de l'invention sont choisies préférentiellement dans le groupe formé par :
- les huiles d'origine animale ou végétale, formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone, en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse telles que l'huile de parléam, les polyoléfines et les esters d'acides carboxyliques liquides ;
- les huiles minérales telles que l'hexadécane, l'isohexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- les huiles de silicone volatiles ou non volatiles.

Les polyoléfines utilisables comme huiles de synthèse sont en particulier les poly-α-oléfines et plus particulièrement celles de type polybutène, hydrogéné ou non, et de préférence polyisobutène, hydrogéné ou non.

Les esters d'acides carboxyliques liquides utilisables comme huiles de synthèse, peuvent être des esters d'acides mono-, di-, tri- ou tétra-carboxyliques. Le nombre total de carbone des esters est généralement supérieur ou égal à 10 et de préférence inférieur à 100 et plus particulièrement inférieur à 80. Ce sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant généralement supérieur ou égal à 10. On peut également utiliser les esters d'acides di- ou tri-carboxyliques en C4-C22 et d'alcools en C1-C22 et les esters d'acides mono-, di-ou tri-carboxyliques et d'alcools di-, tri-, tétra- ou penta-hydroxylés en C2-C26.

Parmi les esters cités ci-dessus, on préfère utiliser les palmitates d'alkyle tels que le palmitate d'éthyle, le palmitate d'isopropyle, le palmitate d'éthyl-2-héxyle, le palmitate de 2-octyldécyle ; les myristates d'alkyle tels que le myristate d'isopropyle, le myristate de butyle, le myristate de cétyle, le myristate de 2-octyldodécyle ; les stéarates d'alkyle tel que le stéarate d'hexyle, le stéarate de butyle, le stéarate d'isobutyle ; les malates d'alkyle tels que le malate de dioctyle ; les laurates d'alkyle tels que le laurate d'hexyle et le laurate de 2-hexyldécyle ; l'isononanoate d'isononyle ; l'octanoate de cétyle.

Les nanoémulsions conformes à l'invention comportent une quantité de phase huileuse (huile et autres corps gras hormis le lipide amphiphile) allant de préférence, de 2 à 40 % en poids par rapport au poids total de la nanoémulsion et plus particulièrement de 4 à 30 % en poids et préférentiellement de 4 à 20 % en poids.

Les nanoémulsions conformes à la présente invention peuvent contenir des solvants, notamment pour améliorer, si nécessaire, la transparence de la composition.

Ces solvants sont choisis de préférence dans le groupe formé par :
- les alcools inférieurs en C₁-C₈ tels que l'éthanol;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.
- les sucres tels que le glucose, le fructose, le maltose, le lactose, le sucrose.

Ces additifs peuvent être utilisés en mélange. Lorsqu'ils sont présents dans la nanoémulsion de l'invention, ils peuvent être utilisés à des concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

Le procédé de préparation d'une nanoémulsion telle que définie ci-dessus consiste à mélanger la phase aqueuse et la phase huileuse, sous agitation vive, à une température ambiante inférieure à 45°C, à effectuer une étape d'homogénéisation haute pression à une pression supérieure à 5.10⁷ Pa et à ajouter le polymère anionique non réticulé à groupement hydrophobe utilisé selon l'invention. Selon un mode préféré de réalisation de l'invention, on effectue ensuite encore une étape d'homogénéisation haute pression à une pression supérieure à 5.10⁷ Pa. L'homogénéisation haute pression est réalisée de préférence à une pression allant de 6.10⁷ à 18.10⁷ Pa. Le cisaillement va de préférence de 2.10⁶ s⁻¹ à 5.10⁸ s⁻¹ et mieux de 1.10⁸ s⁻¹ à 3.10⁸ s⁻¹ (s⁻¹ signifie seconde⁻¹). Un tel procédé permet de réaliser, à température ambiante, des nanoémulsions compatibles avec des composés actifs thermosensibles, et pouvant contenir des huiles et notamment des parfums qui renferment des corps gras, sans les dénaturer.

Les nanoémulsions définies ci-dessus peuvent être utilisées dans tout domaine où ce type de composition est utile. Elles peuvent constituer notamment des compositions à usage topique et en particulier des compositions cosmétiques ou dermatologiques selon le type d'actifs et la quantité de ces actifs qu'elles comportent. Elles peuvent aussi être utilisées comme supports ophtalmiques. Elles peuvent en outre constituer dans le domaine pharmaceutique le support d'une composition pharmaceutique qui peut être administrée par voie orale, parentérale ou transcutanée.

Une telle composition à usage topique, pharmaceutique ou ophtalmique contient un milieu physiologiquement acceptable, c'est-à-dire compatible avec la peau, les muqueuses, le cuir chevelu, les yeux et/ou les cheveux.

L'invention a aussi pour objet un support ophtalmique, caractérisé en ce qu'il contient une nanoémulsion telle que définie précédemment.

L'invention a aussi pour objet une composition pharmaceutique, caractérisée en ce qu'elle contient une nanoémulsion telle que définie précédemment.

Un autre objet de l'invention consiste en une composition cosmétique ou dermatologique, caractérisée en ce qu'elle comprend une nanoémulsion telle que définie précédemment.

Les compositions de l'invention peuvent contenir des adjuvants et notamment des actifs hydrosolubles ou liposolubles, ayant une activité cosmétique ou dermatologique. Les actifs liposolubles sont dans les globules huileux de l'émulsion, tandis que les actifs hydrosolubles sont dans la phase aqueuse de l'émulsion. On peut citer, à titre d'exemples d'actif, les vitamines et leurs dérivés telles que la vitamine E et ses esters tels que l'acétate de vitamine E, la vitamine C et ses esters, les vitamines B, la vitamine A alcool ou rétinol et ses esters tels que le palmitate de vitamine A, la vitamine A acide ou acide rétinoïque et ses dérivés, les provitamines telles que le panthénol, la niacinamide, l'ergocalciférol, les anti-oxydants, les huiles essentielles, les humectants, les filtres solaires, les agents hydratants, les protéines, les céramides et les pseudocéramides. Comme adjuvants, on peut citer aussi les séquestrants, les adoucissants, les matières colorantes (pigments ou colorants) et les parfums.

Comme actifs ophtalmiques, on peut citer par exemple les agents anti-glaucome, tels que le betaxolol ; les antibiotiques tels que l'acyclovir; les antiallergiques ; les agents anti-inflammatoires tels que l'ibuprofène et ses sels, le diclofénac et ses sels, l'indométhacine ; les agents antiviraux.

Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

La nanoémulsion de l'invention peut être par exemple utilisée pour le soin, le traitement, le maquillage de la peau du visage, du corps et/ou du cuir chevelu.

L'invention a donc aussi pour objet l'utilisation cosmétique de la nanoémulsion telle que définie ci-dessus pour le soin, le traitement et/ou le maquillage de la peau.

En outre, la nanoémulsion de l'invention peut aussi être utilisée pour le soin et/ou le traitement des cheveux. Elle permet d'obtenir un dépôt d'huile sur les cheveux, ce qui rend ceux-ci plus brillants, plus résistants au coiffage, sans toutefois les alourdir. Elle permet aussi, en prétraitement d'améliorer les effets de la coloration ou de la permanente.

L'invention a donc aussi pour objet l'utilisation cosmétique de la nanoémulsion telle que définie ci-dessus pour le soin et/ou le traitement des cheveux.

La nanoémulsion selon l'invention permet notamment une bonne hydratation de la peau, des muqueuses et/ou du cuir chevelu et est particulièrement adaptée au traitement de la peau sèche.

Un autre objet de l'invention est donc un procédé cosmétique de soin et/ou d'hydratation de la peau, des muqueuses et/ou du cuir chevelu, caractérisé en ce qu'on applique sur la peau, les muqueuses et/ou le cuir chevelu une nanoémulsion telle que définie ci-dessus.

L'invention porte également sur l'utilisation de la nanoémulsion selon l'invention pour la fabrication d'une composition destinée au traitement de la peau sèche.

Enfin, l'invention porte aussi sur l'utilisation de la nanoémulsion selon l'invention pour la fabrication d'une composition ophtalmologique.

Les exemples qui suivent, permettront de mieux comprendre l'invention, sans toutefois présenter un caractère limitatif. Les quantités indiquées sont en % en poids sauf mention contraire.

| **Exemple 1 : Crème** | | |
|---|---|---|
| A. | Isostéarate de PEG 400 | 4,5 % |
| | Acylgiutamate disodique | 0,5 % |
| | Myristate d'isopropyle | 5 % |
| | Stéarate d'isocétyle | 10 % |
| | | |
| B. | Dipropylène glycol | 10 % |
| | Glycérine | 5 % |
| | Eau distillée | 45 % |
| | | |
| C. | Terpolymère acide méthacrylique/acrylate de méthyle/ Diméthyl m-isopropénylbenzylisocyanate d'alcool béhénylique éthoxylé à 40 DE, en dispersion aqueuse à 24% (soit 0,2 % de matière active) | 0,87 % |
| | Triéthanolamine | 0,14 % |
| | Eau distillée | 18,99 % |

Mode opératoire : La nanoémulsion est préparée à l'homogénéiseur haute-pression à partir des phases A et B. La phase C est introduite sous agitation à la défloculeuse dans la nanoémulsion. L'ensemble est passée à l'homogénéiseur haute-pression dans les mêmes conditions.

On obtient une crème ayant une turbidité de 170 NTU, une viscosité de 3,84 Pa.s (mobile 3, à 200s⁻¹) et un pH d'environ 7. Cette crème s'étale bien sur la peau et est agréable à utiliser.

| **Exemple 2 : Crème** | | |
|---|---|---|
| A. | Isostéarate de PEG 400 | 4,5 % |
| | Acylglutamate disodique | 0,5 % |
| | Myristate d'isopropyle | 5 % |
| | Stéarate d'isocetyle | 10 % |
| | | |
| B. | Dipropylène glycol | 10 % |
| | Glycérine | 5 % |
| | Eau distillée | 37,5 % |
| | | |
| C. | Latex à 30-32% de copolymère acrylate/acrylate modifié par des alcools en C12-24 polyoxyéthylénés (25OE) (Synthalen W2000 de 3V SA) | 0,968 % |
| | Triéthanolamine | 0,194 % |
| | Eau distillée | 26,338 % |

Mode opératoire: La nanoémulsion est préparée à l'homogénéiseur haute-pression à partir des phases A et B. La phase C est introduite sous agitation à la défloculeuse dans la nanoémulsion. L'ensemble est passée à l'homogénéiseur haute-pression dans les mêmes conditions.

On obtient une crème ayant une turbidité de 284 NTU, une viscosité de 0,5 Pa.s (mobile 3, à 200s⁻¹) et un pH d'environ 7. Cette crème s'étale bien sur la peau et est agréable à utiliser.

| **Exemple comparatif :** | | |
|---|---|---|
| A. | Isostéarate de PEG 400 | 4,5 % |
| | Acylglutamate disodique | 0,5 % |
| | Myristate d'isopropyle | 5 % |
| | Stéarate d'isocétyle | 10 % |
| | | |
| B. | Dipropylène glycol | 10 % |
| | Glycérine | 5 % |
| | Eau distillée | 45 % |
| | | |
| C. | Polymère carboxyvinylique (Carbopol 980) | 0,26 % |
| | Triéthanolamine | 0,39 % |
| | Eau distillée | 19,35 % |

L'émulsion est préparée à l'homogénéiseur haute-pression à partir des phases A et B. La phase C est introduite sous agitation à la défloculeuse dans l'émulsion. La composition obtenue a un pH d'environ 7. C'est une crème blanche et non pas une composition transparente.

## Revendications

1. Nanoémulsion huile-dans-eau comportant une phase huileuse dispersée dans une phase aqueuse, dont les globules d'huile ont une taille moyenne inférieure à 100 nm, et comprenant (1) au moins un lipide amphiphile choisi parmi les lipides amphiphiles non toniques, les lipides amphiphiles anioniques et leurs mélanges, le ou les lipides amphiphiles anioniques étant choisis parmi les esters mixtes, d'acide gras ou d'alcool gras, d'acide carboxylique et de glycérol, les citrates d'alkyléther, les alkényl succinates, les esters gras d'acide phosphoriques et leurs mélanges, **caractérisée en ce qu'**elle comprend de plus (2) au moins un polymère anionique non réticulé comportant au moins une chaîne hydrophobe choisi parmi les copolymères d'acide acrylique ou méthacrylique, les copolymères d'acide 2-acrylamido-2-méthylpropane sulfonique et leurs mélanges, et **en ce que** le rapport pondéral de la quantité de phase huileuse sur la quantité de lipide amphiphile va de 2 à 10.

2. Nanoémulsion selon la revendication 1, **caractérisée en ce que** le rapport pondéral de la quantité de phase huileuse sur la quantité de lipide amphiphile va de 2 à 6.

3. Nanoémulsion selon l'une quelconque des revendications précédentes, **caractérisée en ce que** les globules d'huile ont une taille moyenne allant de 20 à 80 nm.

4. Nanoémulsion selon l'une quelconque des revendications précédentes, **caractérisée en ce que** le polymère anionique est hydrosoluble ou hydrodispersible.

5. Nanoémulsion selon l'une quelconque des revendications précédentes, **caractérisée en ce que** la ou les chaînes hydrophobes du polymère anionique sont choisies parmi les chaînes hydrocarbonées linéaires ou ramifiées, saturées ou insaturées, ayant de 6 à 30 atomes de carbone, les groupements divalents cycloaliphatiques et les groupements divalents aromatiques.

6. Nanoémulsion selon l'une quelconque des revendications précédentes, **caractérisée en ce que** la ou les chaînes hydrophobes du polymère anionique sont choisies parmi les chaînes alkyle, arylalkyle, alkylaryle, alcoylène, méthylènedicyclohexyl, isophorone et phénylène.

7. Nanoémulsion selon l'une quelconque des revendications précédentes, **caractérisée en ce que** le polymère anionique a un poids moléculaire allant de 10.000 à 2.000.000.

8. Nanoémulsion selon l'une quelconque des revendications précédentes, **caractérisée en ce que** le polymère anionique est obtenu par copolymérisation d'un monomère (a) choisi parmi les acides carboxyliques à insaturation α, β-éthylénique (monomère a') et l'acide 2-acrylamido 2-méthylpropane sulfonique (monomère a"), avec un monomère (b) à insaturation éthylénique non tensioactif différent de (a) et/ou un monomère (c) à insaturation éthylénique issu de la réaction d'un monomère acrylique à insaturation α,β-monoéthylénique ou d'un monomère isocyanate à insaturation monoéthylénique avec un composant amphiphile non ionique monohydrique ou avec une amine grasse primaire ou secondaire.

9. Nanoémulsion selon l'une quelconque des revendications précédentes, **caractérisée en ce que** le polymère anionique est un terpolymère acrylique obtenu à partir de (a) un acide carboxylique à insaturation α,β-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique.

10. Nanoémulsion selon l'une quelconque des revendications 1 à 8, **caractérisée en ce que** le polymère anionique est choisi parmi le terpolymère acide acrylique/acrylate d'ethyle/acrylate d'alkyle, le copolymère acrylates/steareth-20 methacrylate, le terpolymère acide (meth)acrylique / acrylate d'éthyle /methacrylate de béhényle oxyéthyléné (25 OE), le copolymère acide acrylique/itaconate de mono-cétyle oxyéthyléné (20 OE), le copolymère acide acrylique/itaconate de mono-stéaryle oxyéthyléné (20 OE), le copolymère acrylates/acrylate modifié par des alcools en C12-C24 polyoxyéthylénés (25 OE), le terpolymère acide methacrylique/acrylate de méthyle/diméthylmétaisopropényl benzylisocyanate d'alcool béhénylique éthoxylé, et leurs mélanges.

11. Nanoémulsion selon l'une quelconque des revendications précédentes, **caractérisée en ce que** la quantité de polymère(s) anionique(s) va de 0.1 à 10 % en poids par rapport au poids total de la composition.

12. Nanoémulsion selon l'une quelconque des revendications précédentes, **caractérisée en ce que** le lipide amphi-phile non ionique est choisi parmi :
1/ les tensioactifs siliconés,
2/ les lipides amphiphiles liquides à température inférieure ou égale à 45°C choisis parmi les esters d'au moins un polyol et d'au moins un acide gras comportant au moins une chaîne alkyle en C₈-C₂₂, saturée ou non saturée, linéaire ou ramifiée,
3/ les esters d'acide gras et de sucre et les éthers d'alcool gras et de sucre,
4/ les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés,
5/ les copolymères blocs d'oxyde d'éthylène (A) et d'oxyde de propylène (B), et leurs mélanges.

13. Nanoémulsion selon l'une quelconque des revendications précédentes, **caractérisée en ce que** la quantité de lipide amphiphile va de 0,2 à 15 % en poids par rapport au poids total de la composition.

14. Nanoémulsion selon l'une quelconque des revendications précédentes,
**caractérisée en ce qu'**elle comprend en outre au moins un lipide amphiphile anionique additionnel choisi parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique;
- les sels de sodium de l'acide phosphatidique ;
- les phospholipides ;
- les dérivés alkylsulfoniques notamment de formule : 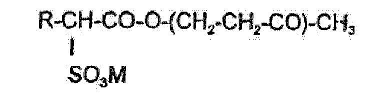
dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium.

15. Nanoémulsion selon la revendication précédente, **caractérisée en ce que** le lipide amphiphile additionnel anionique est présent en une quantité allant de 0,01 à 10 % en poids par rapport au poids total de la composition.

16. Nanoémulsion selon l'une quelconque des revendications précédentes, **caractérisée en ce que** la quantité de phase huileuse va de 2 à 40 % en poids par rapport au poids total de la composition.

17. Nanoémulsion selon l'une quelconque des revendications précédentes, **caractérisée en ce qu'**elle a une turbidité allant de 60 à 600 NTU.

18. Composition cosmétique ou dermatologique, **caractérisée en ce qu'**elle comprend une nanoémulsion selon l'une quelconque des revendications précédentes.

19. Support ophtalmique, **caractérisé en ce qu'**il contient une nanoémulsion selon l'une quelconque des revendications 1 à 17.

20. Composition pharmaceutique, **caractérisée en ce qu'**elle contient une nanoémulsion selon l'une quelconque des revendications 1 à 17.

21. Utilisation cosmétique de la nanoémulsion selon l'une quelconque des revendications 1 à 17, pour le soin, le traitement et/ou le maquillage de la peau.

22. Utilisation cosmétique de la nanoémulsion selon l'une quelconque des revendications 1 à 17, pour le soin et/ou le traitement des cheveux.

23. Procédé cosmétique de soin et/ou d'hydratation de la peau, des muqueuses et/ou du cuir chevelu, **caractérisé en ce qu'**on applique sur la peau, les muqueuses et/ou le cuir chevelu, une nanoémulsion selon l'une quelconque des revendications 1 à 17 ou une composition cosmétique selon la revendication 18.

24. Utilisation de la nanoémulsion selon l'une quelconque des revendications 1 à 17, pour la fabrication d'une composition destinée au traitement de la peau sèche.

25. Utilisation de la nanoémulsion selon l'une quelconque des revendications 1 à 17, pour la fabrication d'une composition ophtalmologique.

26. Procédé pour épaissir une nanoémulsion huile-dans-eau ayant des globules d'huile dont la taille moyenne est inférieure à 100 nm, consistant à ajouter à ladite nanoémulsion, un polymère anionique non réticulé à groupement hydrophobe choisi parmi les copolymères d'acide acrylique ou méthacrylique, les copolymères d'acide 2-acrylamido-2-méthylpropane sulfonique et leurs mélanges.

## Claims

1. Oil-in-water nanoemulsion comprising an oily phase dispersed in an aqueous phase, the oil globules of which have an average size of less than 100 nm, and comprising (1) at least one amphiphilic lipid chosen from nonionic amphiphilic lipids, anionic amphiphilic lipids and their mixtures, the anionic amphiphilic lipid or lipids being chosen from mixed esters of fatty acid and of fatty alcohol, of carboxylic acid and of glycerol, alkyl ether citrates, alkenyl succinates, fatty esters of phosphoric acid and their mixtures, **characterized in that** it also comprises (2) at least one non crosslinked anionic polymer comprising at least one hydrophobic chain, said polymer being chosen from acrylic or methacrylic acid copolymers, 2-acrylamido-2-methylpropanesulphonic acid copolymers and their mixtures, and **in that** the ratio by weight of the amount of oily phase to the amount of amphiphilic lipid ranges from 2 to 10.

2. Nanoemulsion according to Claim 1, **characterized in that** the ratio by weight of the amount of oily phase to the amount of amphiphilic lipid ranges from 2 to 6.

3. Nanoemulsion according to either one of the preceding claims, **characterized in that** the oil globules have a mean size ranging from 20 to 80 nm.

4. Nanoemulsion according to any one of the preceding claims, **characterized in that** the anionic polymer is water-soluble or water-dispersible.

5. Nanoemulsion according to any one of the preceding claims, **characterized in that** the hydrophobic chain or chains of the anionic polymer are chosen from saturated or unsaturated and linear or branched hydrocarbonaceous chains having from 6 to 30 carbon atoms, divalent cycloaliphatic groups and divalent aromatic groups.

6. Nanoemulsion according to any one of the preceding claims, **characterized in that** the hydrophobic chain or chains of the anionic polymer are chosen from alkyl, arylalkyl, alkylaryl, alkylene, methylene-dicyclohexyl, isophorone and phenylene chains.

7. Nanoemulsion according to any one of the preceding claims, **characterized in that** the anionic polymer has a molecular weight ranging from 10 000 to 2 000 000.

8. Nanoemulsion according to any one of the preceding claims, **characterized in that** the anionic polymer is obtained by copolymerization of a monomer (a) chosen from carboxylic acids comprising α,β-ethylenic unsaturation (monomer a') and 2-acrylamido-2-methylpropanesulphonic acid (monomer a") with a non-surface-active monomer (b) comprising ethylenic unsaturation other than (a) and/or a monomer (c) comprising ethylenic unsaturation resulting from the reaction of an acrylic monomer comprising α,β-monoethylenic unsaturation or of an isocyanate monomer comprising monoethylenic unsaturation with a monohydric nonionic amphiphilic component or with a primary or secondary fatty amine.

9. Nanoemulsion according to any one of the preceding claims, **characterized in that** the anionic polymer is an acrylic terpolymer obtained from (a) a carboxylic acid comprising α,β-ethylenic unsaturation, (b) a non-surface-active monomer comprising ethylenic unsaturation other than (a), and (c) a nonionic urethane monomer which is the reaction product of a monohydric nonionic amphiphilic compound with an isocyanate comprising monoethylenic unsaturation.

10. Nanoemulsion according to any one of Claims 1 to 9, **characterized in that** the anionic polymer is chosen from the acrylic acid/ethyl acrylate/alkyl acrylate terpolymer, the acrylates/steareth-20 methacrylate copolymer, the (meth)acrylic acid/ethyl acrylate/oxyethylenated (25 EO) behenyl methacrylate terpolymer, the acrylic acid/oxyethylenated (20 EO) monocetyl itaconate copolymer, the acrylic acid/oxyethylenated (20 EO) monostearyl itaconate copolymer, the acrylates/acrylate modified by polyoxyethylenated (25 EO) C₁₂-C₂₄ alcohols copolymer, the methacrylic acid/methyl acrylate/dimethyl-meta-isopropenylbenzyl isocyanate of ethoxylated behenyl alcohol terpolymer, and their mixtures.

11. Nanoemulsion according to any one of the preceding claims, **characterized in that** the amount of anionic polymer(s) ranges from 0.1 to 10% by weight with respect to the total weight of the composition.

12. Nanoemulsion according to any one of the preceding claims, **characterized in that** the nonionic amphiphilic lipid is chosen from:
1) silicone surfactants,
2) amphiphilic lipids which are liquid at a temperature of less than or equal to 45°C chosen from esters of at least one polyol and of at least one fatty acid comprising at least one saturated or unsaturated and linear or branched C₈-C₂₂ alkyl chain,
3) esters of fatty acid and of sugar and ethers of fatty alcohol and of sugar,
4) surfactants which are solid at a temperature of less than or equal to 45°C chosen from glycerol fatty esters, sorbitan fatty esters and oxyethylenated sorbitan fatty esters, ethoxylated fatty ethers and ethoxylated fatty esters,
5) block copolymers of ethylene oxide (A) and of propylene oxide (B),
and their mixtures.

13. Nanoemulsion according to any one of the preceding claims, **characterized in that** the amount of amphiphilic lipid ranges from 0.2 to 15% by weight with respect to the total weight of the composition.

14. Nanoemulsion according to any one of the preceding claims, **characterized in that** it moreover comprises at least one additional anionic amphiphilic lipid chosen from:
- alkaline salts of dicetyl and dimyristyl phosphate;
- alkaline salts of cholesterol sulphate;
- alkaline salts of cholesterol phosphate;
- lipoamino acids and their salts, such as mono- and disodium acylglutamates, for instance the disodium salt of N-stearoyl-L-glutamic acid;
- sodium salts of phosphatidic acid;
- phospholipids;
- alkylsulphonic derivatives, in particular of formula: 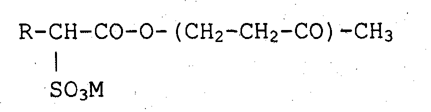
in which R represents C₁₆-C₂₂ alkyl radicals, in particular the C₁₆H₃₃ and C₁₈H₃₇ radicals taken as a mixture or separately, and M is an alkali metal or an alkaline earth metal, such as sodium.

15. Nanoemulsion according to the preceding claim, **characterized in that** the additional anionic amphiphilic lipid is present in an amount ranging from 0.01 to 10% by weight with respect to the total weight of the composition.

16. Nanoemulsion according to any one of the preceding claims, **characterized in that** the amount of oily phase ranges from 2 to 40% by weight with respect to the total weight of the composition.

17. Nanoemulsion according to any one of the preceding claims, **characterized in that** it has a turbidity ranging from 60 to 600 NTU.

18. Cosmetic or dermatological composition, **characterized in that** it comprises a nanoemulsion according to any one of the preceding claims.

19. Ophthalmic vehicle, **characterized in that** it comprises a nanoemulsion according to any one of Claims 1 to 17.

20. Pharmaceutical composition, **characterized in that** it comprises a nanoemulsion according to any one of Claims 1 to 17.

21. Cosmetic use of the nanoemulsion according to any one of Claims 1 to 17 for caring for, treating and/or making up the skin.

22. Cosmetic use of the nanoemulsion according to any one of Claims 1 to 17 for caring for and/or treating the hair.

23. Cosmetic process for caring for and/or moisturizing the skin, mucous membranes and/or scalp, **characterized in that** a nanoemulsion according to any one of Claims 1 to 17 or a cosmetic composition according to Claim 18 is applied to the skin, mucous membranes and/or scalp.

24. Use of the nanoemulsion according to any one of Claims 1 to 17 in the manufacture of a composition intended for the treatment of dry skin.

25. Use of the nanoemulsion according to any one of Claims 1 to 17 in the manufacture of an ophthalmological composition.

26. Process for thickening an oil-in-water nanoemulsion having oil globules with a number-average size of less than 100 nm, which consists in adding to the said nanoemulsion a non crosslinked anionic polymer comprising at least one hydrophobic chain, said polymer being chosen from acrylic or methacrylic acid copolymers, 2-acrylamido-2-methylpropanesulphonic acid copolymers and their mixtures.

## Patentansprüche

1. Öl-in-Wasser-Nanoemulsion, die eine in einer wässerigen Phase dispergierte Ölphase aufweist, wobei die mittlere Größe der Ölkügelchen unter 100 nm liegt, und die (1) mindestens ein amphiphiles Lipid enthält, das unter nichtionischen amphiphilen Lipiden, anionischen amphiphilen Lipiden und deren Gemischen ausgewählt ist, wobei das oder die anionischen amphiphilen Lipide unter den gemischten Estern einer Fettsäure oder eines Fettalkohols, einer Carbonsäure und Glycerin, Alkylethercitraten, Alkenylsuccinaten, Fettsäureestern von Phosphorsäure und deren Gemischen ausgewählt sind, **dadurch gekennzeichnet, dass** sie ferner (2) mindestens ein unvernetzt anionisches Polymer, das mindestens eine hydrophobe Gruppe aufweist, enthält, wobei das unvernetzt anionisches Polymer unter den Copolymeren von Acrylsäure oder Methacrylsäure, den Copolymeren von 2-Acrylamido-2-methylpropansulfonsäure und deren Gemischen ausgewählt ist, sowie dadurch, dass das Gewichtsverhältnis der Menge der Ölphase zu der Menge des amphiphilen Lipids im Bereich von 2 bis 10 liegt.

2. Nanoemulsion nach Anspruch 1, **dadurch gekennzeichnet, dass** das Gewichtsverhältnis der Menge der Ölphase zu der Menge des amphiphilen Lipids im Bereich von 2 bis 6 liegt.

3. Nanoemulsion nach einem der vorhergehenden Ansprüche, **dadurch gekennzeichnet, dass** die mittlere Größe der Ölkügelchen im Bereich von 20 bis 80 nm liegt.

4. Nanoemulsion nach einem der vorhergehenden Ansprüche, **dadurch gekennzeichnet, dass** das anionische Polymer wasserlöslich oder in Wasser dispergierbar ist.

5. Nanoemulsion nach einem der vorhergehenden Ansprüche, **dadurch gekennzeichnet, dass** die hydrophobe Gruppe oder die hydrophoben Gruppen des anionischen Polymers unter geradkettigen oder verzweigten, gesättigten oder ungesättigten Kohlenwasserstoffgruppen mit 6 bis 30 Kohlenstoffatomen, cycloaliphatischen zweiwertigen Gruppen und aromatischen zweiwertigen Gruppen ausgewählt sind.

6. Nanoemulsion nach einem der vorhergehenden Ansprüche, **dadurch gekennzeichnet, dass** die hydrophobe Gruppe oder die hydrophoben Gruppen des anionischen Polymers unter Alkyl, Arylalkyl, Alkylaryl, Alkylen, Methylendicyclohexyl, Isophoron und Phenylen ausgewählt sind.

7. Nanoemulsion nach einem der vorhergehenden Ansprüche, **dadurch gekennzeichnet, dass** das anionische Polymer ein Molekulargewicht im Bereich von 10 000 bis 2 000 000 aufweist.

8. Nanoemulsion nach einem der vorhergehenden Ansprüche, **dadurch gekennzeichnet, dass** das anionische Polymer durch Copolymerisation eines Monomers (a), das unter α,β-ethylenisch ungesättigten Carbonsäuren (Monomer a') und 2-Acrylamido-2-methylpropansulfonsäure (Monomer a") ausgewählt ist, mit einem nicht grenzflächenaktiven ethylenisch ungesättigten Monomer (b), das von (a) verschieden ist, und/oder einem ethylenisch ungesättigten Monomer (c) erhalten wird, das aus der Umsetzung eines α,β-monoethylenisch ungesättigten Acrylmonomers oder eines monoethylenisch ungesättigten Isocyanat-Monomers mit einer nichtionischen amphiphilen Komponente mit einer Hydroxygruppe oder mit einem primären oder sekundären Fettamin stammt.

9. Nanoemulsion nach einem der vorhergehenden Ansprüche, **dadurch gekennzeichnet, dass** das anionische Polymer ein Acrylterpolymer ist, das aus (a) einer α,β-monoethylenisch ungesättigten Carbonsäure, (b) einem nicht grenzflächenaktiven ethylenisch ungesättigten Monomer, das von (a) verschieden ist, und (c) einem nichtionischen Urethanmonomer, das das Produkt der Umsetzung einer nichtionischen amphiphilen Verbindung mit einer Hydroxygruppe mit einem monoethylenisch ungesättigten Isocyanat ist, hergestellt ist.

10. Nanoemulsion nach einem der Ansprüche 1 bis 8, **dadurch gekennzeichnet, dass** das anionische Polymer unter einem Terpolymer von Acrylsäure, Ethylacrylat und Alkylacrylat, einem Copolymer von Acrylaten und Steareth-20 methacrylat, einem Terpolymer von (Meth)-acrylsäure, Ethylacrylat und ethoxyliertem Behenylmethacrylat (25 EO), einem Copolymer von Acrylsäure und ethoxyliertem Monocetylitaconat (20 EO), einem Copolymer von Acrylsäure und ethoxyliertem Monostearylitaconat (20 EO), einem Copolymer von Acrylaten und mit polyethoxylierten C₁₂₋₂₄-Alkoholen modifiziertem Acrylat (25 EO), einem Terpolymer von Methacrylsäure, Methylacrylat und Dimethyl-m-isopropenylbenzylisocyanat von ethoxyliertem Behenylalkohol und den Gemischen dieser Verbindungen ausgewählt ist.

11. Nanoemulsion nach einem der vorhergehenden Ansprüche, **dadurch gekennzeichnet, dass** der Mengenanteil des anionischen Polymers (der anionischen Polymere) im Bereich von 0,1 bis 10 Gew.-%, bezogen auf das Gesamtgewicht der Zusammensetzung, liegt.

12. Nanoemulsion nach einem der vorhergehenden Ansprüche, **dadurch gekennzeichnet, dass** das nichtionische amphiphile Lipid ausgewählt ist unter:
1/ siliconhaltigen grenzflächenaktiven Stoffen,
2/ amphiphilen Lipiden, die bei einer Temperatur von kleiner oder gleich 45 °C flüssig sind und die unter den Estern von mindestens einem Polyol und mindestens einer Fettsäure, die mindestens eine gesättigte oder nicht gesättigte, geradkettige oder verzweigte C₈₋₂₂-Alkylgruppe aufweist, ausgewählt sind,
3/ den Estern einer Fettsäure und eines Zuckers sowie den Ethern eines Fettalkohols und eines Zuckers,
4/ grenzflächenaktiven Stoffen, die bei einer Temperatur von kleiner oder gleich 45 °C fest sind und die unter Glycerinfettsäureestern, Sorbitanfettsäureestern und ethoxylierten Sorbitanfettsäureestern, ethoxylierten Fettethern und ethoxylierten Fettestern ausgewählt sind,
5/ Blockcopolymeren von Ethylenoxid (A) und Propylenoxid (B), und den Gemischen dieser Verbindungen.

13. Nanoemulsion nach einem der vorhergehenden Ansprüche, **dadurch gekennzeichnet, dass** der Mengenanteil des amphiphilen Lipids im Bereich von 0,2 bis 15 Gew.-%, bezogen auf das Gesamtgewicht der Zusammensetzung, liegt.

14. Nanoemulsion nach einem der vorhergehenden Ansprüche, **dadurch gekennzeichnet, dass** sie ferner mindestens ein zusätzliches anionisches amphiphiles Lipid umfasst, das ausgewählt ist unter:
- Alkalisalzen von Dicetylphosphat und Dimyristylphosphat,
- Alkalisalzen von Cholesterylsulfat,
- Alkalisalzen von Cholesterylphosphat,
- Lipoaminosäuren und ihren Salzen, wie Mononatriumacylglutamat und Dinatriumacylglutamat, wie dem Dinatriumsalz von N-Stearoyl-L-glutaminsäure,
- den Natriumsalzen von Phosphatidsäure,
- Phospholipiden und
- Alkylsulfonsäurederivaten und insbesondere den Verbindungen der Formel: 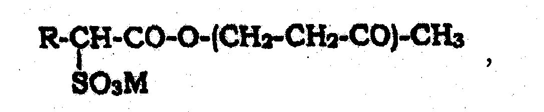
worin bedeuten:
- R C₁₈₋₂₂-Alkylgruppen und insbesondere die Gruppen C₁₆H₃₃ und C₁₈H₃₇, die im Gemisch oder getrennt voneinander verwendet werden und
- M ein Alkalimetall oder ein Erdalkalimetall, wie Natrium.

15. Nanoemulsion nach dem vorhergehenden Anspruch, **dadurch gekennzeichnet, dass** das zusätzliche anionische amphiphile Lipid in einem Mengenanteil von 0,01 bis 10 Gew.-%, bezogen auf das Gesamtgewicht der Zusammensetzung, vorliegt.

16. Nanoemulsion nach einem der vorhergehenden Ansprüche, **dadurch gekennzeichnet, dass** der Mengenanteil der Ölphase im Bereich von 2 bis 40 Gew.-%, bezogen auf das Gesamtgewicht der Zusammensetzung, liegt.

17. Nanoemulsion nach einem der vorhergehenden Ansprüche, **dadurch gekennzeichnet, dass** sie eine Trübung von 60 bis 600 NTU aufweist.

18. Kosmetische oder dermatologische Zusammensetzung, **dadurch gekennzeichnet, dass** sie eine Nanoemulsion nach einem der vorhergehenden Ansprüche enthält.

19. Ophthalmischer Träger, **dadurch gekennzeichnet, dass** er eine Nanoemulsion nach einem der Ansprüche 1 bis 17 enthält.

20. Pharmazeutische Zusammensetzung, **dadurch gekennzeichnet, dass** sie eine Nanoemulsion nach einem der Ansprüche 1 bis 17 enthält.

21. Kosmetische Verwendung der Nanoemulsion nach einem der Ansprüche 1 bis 17 zur Pflege, zur Behandlung und/ oder zum Schminken der Haut.

22. Kosmetische Verwendung der Nanoemulsion nach einem der Ansprüche 1 bis 17 zur Pflege und/oder zur Behandlung der Haare.

23. Kosmetisches Verfahren zur Pflege und/oder zur Hydratisierung der Haut, der Schleimhäute und/oder der Kopfhaut, **dadurch gekennzeichnet, dass** eine Nanoemulsion nach einem der Ansprüche 1 bis 17 oder eine kosmetische Zusammensetzung nach Anspruch 18 auf die Haut, die Schleimhäute und/ oder die Kopfhaut aufgebracht wird.

24. Verwendung der Nanoemulsion nach einem der Ansprüche 1 bis 17 zur Herstellung einer Zusammensetzung, die zur Behandlung von trockener Haut vorgesehen ist.

25. Verwendung der Nanoemulsion nach einem der Ansprüche 1 bis 17 zur Herstellung einer ophthalmologischen Zusammensetzung.

26. Verfahren zum Verdicken einer Öl-in-Wasser-Nanoemulsion mit Ölkügelchen, deren mittlere Größe unter 100 nm liegt, das darin besteht, ein unvernetzt anionisches Polymer, das mindestens eine hydrophobe Gruppe aufweist, wobei das unvernetzt anionisches Polymer unter den Copolymeren von Acrylsäure oder Methacrylsäure, den Copolymeren von 2-Acrylamido-2-methylpropansulfonsäure und deren Gemischen ausgewählt ist, zu der Nanoemulsion zu geben.
